# EUROPEAN PATENT APPLICATION

(11) **EP 4 383 269 A1**
(43) Date of publication of application: **12.06.2024**
(21) Application number: 22211637.8
(22) Date of filing: 06.12.2022
(51) Int. Cl.: G16H 20/30, B26B 19/38, G16H 40/63

(54) **DETERMINING A LOCATION OF A PERSONAL CARE DEVICE**

(71) Applicant: Koninklijke Philips N.V., 5656 AG Eindhoven (NL)
(72) Inventor: FLINSENBERG, Ingrid Christina Maria, 5656AG Eindhoven (NL); VAN ZUTPHEN, Martijn, 5656AG Eindhoven (NL)
(74) Representative: Philips Intellectual Property & Standards

(57) **Abstract**

According to an aspect, there is provided a computer-implemented method (100) for determining a location of a personal care device relative to a body part of a subject, the method comprising receiving (102) an indication of an intended treatment area of the body part to be treated using the personal care device; determining (104), based on the received indication, geometrical properties of the intended treatment area; receiving (106), from a first sensor associated with the personal care device, first sensor data associated with a location of the personal care device relative to the body part acquired during a treatment session performed using the personal care device; predicting (108), based on the first sensor data received from the first sensor, a sequence of locations of the personal care device relative to the body part; and modifying (110) the sequence of predicted locations based on the determined geometrical properties of the intended treatment area.

## Description

### FIELD OF THE INVENTION

The invention relates to the localization of a personal care device and, more particularly, to determining a location of a personal care device relative to a body part of a subject.

### BACKGROUND OF THE INVENTION

Personal care devices are used by people to perform personal care activities, such as hair care activities (e.g., shaving or trimming), skin treatment activities (e.g., skin brushing) and oral care activities (e.g., toothbrushing).

It can be beneficial to be determine the location of a personal care device while a user is performing a personal care activity, for example to enable an assessment of a level of completion of a personal care activity to be made. However, some systems that enable the location of a personal care device to be determined can require a series of complex sensors. In some systems, cameras may be required to accurately determine the location of the personal care device, but this can lead to privacy concerns, particularly when the personal care device is used in a bathroom environment, for example.

In systems that do not use cameras, movement of the personal care device may be detected, but it can be difficult to determine which body part is being treated, and whether the entire body part is being treated, or just a portion of the body part.

There is therefore a desire for system that enables the location of a personal care device to be determined relative to a body part to be treated.

### SUMMARY OF THE INVENTION

A need has been identified to determine the location of a personal care device relative to a body part being treated that overcomes the above-mentioned issues. The inventors of the present disclosure have recognised that data obtained from a sensor (e.g., an inertial measurement unit, or IMU) associated within the personal care device can provide an indication of the overall motion made by the personal care device during a personal care activity, and that this motion can be modified (e.g., scaled) based on knowledge of the body part - and, more specifically, the area of the body part - being treated during the personal care activity.

According to a first specific aspect, there is provided a computer-implemented method for determining a location of a personal care device relative to a body part of a subject, the method comprising receiving an indication of an intended treatment area of the body part to be treated using the personal care device; determining, based on the received indication, geometrical properties of the intended treatment area; receiving, from a first sensor associated with the personal care device, first sensor data associated with a location of the personal care device relative to the body part acquired during a treatment session performed using the personal care device; predicting, based on the first sensor data received from the first sensor, a sequence of locations of the personal care device relative to the body part; and modifying the sequence of predicted locations based on the determined geometrical properties of the intended treatment area.

In this way, a single sensor associated with the personal care device can be used to obtain data indicative of the device motion, and this can be modified based on knowledge of where the device was being used when the measurements were obtained. Multiple sensors are not therefore required, and the localization can be achieved without the use of cameras. Moreover, if the personal care device is also capable of measuring data indicative of the performance quality of the personal care activity (e.g., speed of the treatment, pressure applied, and the like), then this data can also be associated with a particular region of the body part being treated, leading to a more meaningful assessment of the quality of the treatment at different areas of the body part.

In some embodiments, modifying the sequence of predicted locations may comprise rescaling the sequence of predicted locations based on the determined geometrical properties of the intended treatment area.

Modifying the sequence of predicted locations may comprise, responsive to determining that the sequence of predicted locations includes one or more locations that are outside of the intended treatment area, modifying the sequence of predicted locations such that the sequence of modified locations is within the intended treatment area.

The method may, in some embodiments, further comprise projecting the sequence of modified locations onto a representation of the intended treatment area.

In some embodiments, the method may further comprise receiving, during a treatment session, from a second sensor associated with the personal care device, second sensor data relating to a treatment parameter associated with the personal care device. The method may further comprise generating, for delivery to a recipient device, feedback relating to the treatment session, based on the first sensor data received from the first sensor and the second sensor data received from the second sensor.

The method may further comprise providing the generated feedback for presentation to a user on a representation of the body part.

In some embodiments, the indication of the intended treatment area of the body part may comprise an indication of an area to be treated, or an area not to be treated, on a segmented representation of the body part displayed to the user.

The indication of the intended treatment area of the body part may comprise an image of an intended treatment style or pattern on the body part. The step of determining the geometrical properties of the intended treatment area may comprise determining the geometrical properties from the intended treatment style or pattern.

The method may further comprise applying a weight to each predicted location in the sequence of predicted locations based on a distance of the predicted location from the intended treatment area. The step of modifying the sequence of predicted locations may be further based on the applied weights.

According to a second specific aspect, there is provided personal care device comprising a first sensor configured to measure first sensor data associated with a location of the personal care device relative to a body part to be treated. The personal care device further comprises a processor configured to receive an indication of an intended treatment area of the body part to be treated using the personal care device; determine, based on the received indication, geometrical properties of the intended treatment area; receive first sensor data from the first sensor during a treatment session performed using the personal care device; predict, based on the first sensor data received from the first sensor, a sequence of locations of the personal care device relative to the body part; and modify the sequence of predicted locations based on the determined geometrical properties of the intended treatment area.

In some embodiments, the first sensor of the personal care device may comprise a sensor selected from a group comprising: an inertial measurement unit, IMU, an accelerometer, a displacement sensor, and a proximity sensor.

The personal care device may further comprise a second sensor configured to measure a treatment parameter associated with the personal care device. The processor may be configured to receive second sensor data from the second sensor during the treatment session; and
generate, for delivery to a recipient device, feedback relating to the treatment session based on first sensor data received from the first sensor and second sensor data received from the second sensor.

In some embodiments, the processor of the personal care device may be configured to modify the sequence of predicted locations by rescaling the sequence of predicted locations such that the sequence of modified locations is within the intended treatment area.

According to a third specific aspect, there is provided a system for determining a location of a personal care device relative to a body part of a subject, the system comprising a personal care device; a first sensor associated with the personal care device, the first sensor being configured to generate first sensor data associated with a location of the personal care device relative to the body part; a processor configured to receive an indication of an intended treatment area of the body part to be treated using the personal care device; determine, based on the received indication, geometrical properties of the intended treatment area; receive, from the first sensor, first sensor data generated by the first sensor during a treatment session performed using the personal care device; predict, based on the first sensor data received from the first sensor, a sequence of locations of the personal care device relative to the body part; and modify the sequence of predicted locations based on the determined geometrical properties of the intended treatment area.

According to a fourth specific aspect, there is provided a computer program product comprising a non-transitory computer-readable medium, the computer-readable medium having computer-readable code embodied therein, the computer readable code being configured such that, on execution by a suitable computer or processor, the computer or processor is caused to perform steps of the methods disclosed herein.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a flowchart of an example of a method of determining a location of a personal care device relative to a body part of a subject;
Fig. 2 is a flowchart of a further example of a method of determining a location of a personal care device relative to a body part of a subject;
Fig. 3 is an illustration of an example of a user interface;
Fig. 4 is a flowchart of a further example of a method of determining a location of a personal care device relative to a body part of a subject;
Fig. 5 is a schematic illustration of an example of a personal care device;
Fig. 6 is a schematic illustration of an example of a system for determining a location of a personal care device relative to a body part of a subject; and
Fig. 7 is a schematic illustration of an example of a processor in communication with a machine-readable medium.

### DETAILED DESCRIPTION OF THE EMBODIMENTS

According to various embodiments disclosed herein, there is provided a mechanism by which data indicative of how a personal care device is moved during a personal care activity can be used to accurately determine the location of the personal care device relative to a body part.

As used herein, the term "personal care device" is intended to refer to any device or instrument that may be used to perform a personal care activity. A personal care device may, for example, include a hair cutting device such as a hair trimmer or a shaving device, a skin treatment device such as a facial cleansing brush, a skin rejuvenation device or an intense pulsed light (IPL) device, an oral care device such as a power toothbrush or an air flossing device, or the like. The term "body part" as used herein, is intended to refer to any part of the body of a human that may be treated using a personal care device. For example, a shaving device (i.e., the personal care device) may be used to perform a shaving activity (i.e., the personal care activity) on the head (i.e., the body part) of the subject. In another example, an intense pulsed light (IPL) device (i.e., the personal care device) may be used to perform a hair removal activity (i.e., the personal care activity) on the leg (i.e., the body part) of the subject.

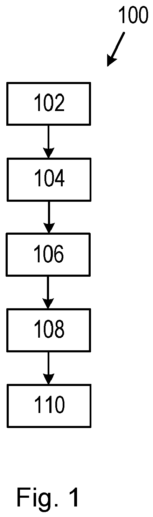

According to a first aspect, the present invention provides a method. Referring to the drawings, a method according to various embodiments is described with reference to Figs. 1 to 3. Fig. 1 is a flowchart of an example of a method 100 for determining a location of a personal care device relative to a body part of a subject. The subject may, for example, be the user of the personal care device. The method 100 may comprise a computer-implemented method, and steps of the method may be performed using one or more processors or processing apparatuses. The method 100 comprises, at step 102, receiving an indication of an intended treatment area of the body part to be treated using the personal care device. At step 104, the method 100 comprises determining, based on the received indication, geometrical properties (e.g., dimensions) of the intended treatment area. The method comprises, at step 106, receiving, from a first sensor associated with the personal care device, first sensor data associated with a location of the personal care device relative to the body part acquired during a treatment session performed using the personal care device. At step 108, the method comprises predicting, based on the first sensor data received from the first sensor, a sequence of locations of the personal care device relative to the body part. At step 110, the method comprises modifying the sequence of predicted locations based on the determined geometrical properties of the intended treatment area.

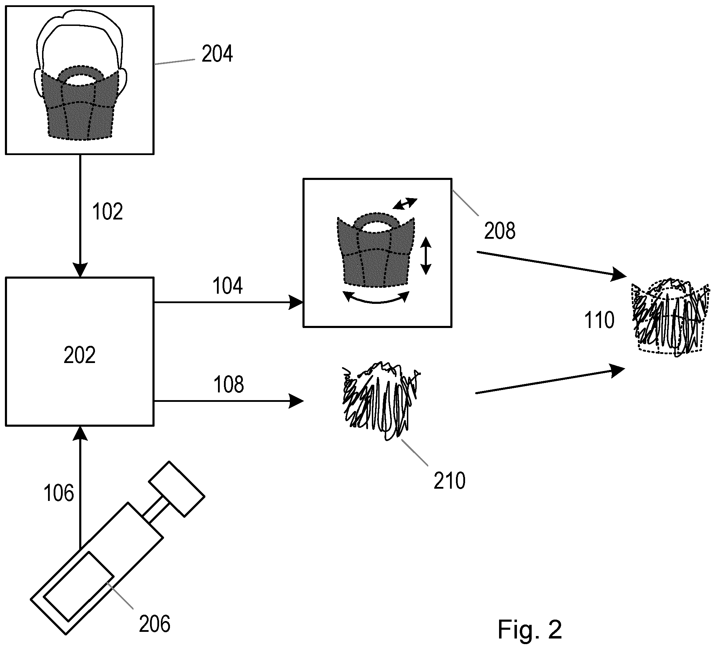

The steps of the method 100 are described in greater detail with reference the example shown in Fig. 2, in which a processor 202 is used to perform the various steps. While a single processor 202 is shown in this example, it will be appreciated that multiple processors may be used, and different steps may be performed by different processing resources, which may be located in the same location or in different locations. The example shown in Fig. 2 relates to a shaving activity, where a shaving device is used to cut (i.e., shave) hair on the head (e.g., face) of a subject.

The processor 202 receives (step 102) an indication of an intended treatment area of the body part to be treated using the personal care device. In some embodiments, the indication of an intended treatment area may be received via a user input (e.g., from a user). Such a user input 204 may be provided via a user interface, for example a user interface associated with a computing device such as a desktop computer, a tablet computer, a smartphone, a wearable device, and interactive mirror, or the like. An interactive mirror, sometimes referred to as a smart mirror, is a unit which, in addition to functioning as a mirror to show a user his or her reflection, is also capable of displaying information to the user. Information, such as text, images and videos, may be displayed on a display portion of the interactive mirror which may, for example, be positioned behind a mirrored (or partially mirrored) panel or a mirrored (or partially mirrored) surface. In this way, the display screen, or portions thereof, may be visible through the mirror portion, so that a user is able to simultaneously view their reflection and information presented on the display screen.

A user may, for example, provide a textual input, by typing in text describing the intended treatment area (e.g., chin), or selecting an intended treatment area from a list of possible treatment areas. In other examples, a user may provide an indication by selecting the intended treatment area from a representation (e.g., an image) of the body part displayed to them. For example, the indication of the intended treatment area of the body part may comprise an indication of an area to be treated, or an area not to be treated, on a segmented representation of the body part displayed to the user. The segmented representation may be presented to a user on a display screen of a computing device (e.g., the user's smartphone), and the user may indicate the intended treatment area or areas by selecting the relevant segments or regions in the representation. An indication of the intended treatment area may be provided as a result of the user indicating an area or areas that are not to be treated. For example, if a user indicates that particular areas are not intended to be treated, it may be understood that any areas that have not been selected are intended to be treated. In an example where a user is performing a shaving activity, the user may indicate areas on the face of the subject where it is intended that hair should remain, and the processor 202 may determine that the other areas are intended to be shaved.

In some embodiments, the indication of the intended treatment area of the body part may comprise an image and intended treatment style or pattern on the body part. For example, a user may select, from a plurality of options, a treatment style (e.g., a facial hair style) that they would like to achieve. In other examples, the user may upload an image of the intended treatment style or pattern, such as an image of a person whose body part has been treated according to the intended treatment style or pattern. For example, the user may upload an image of a person having a facial hair style that they would like to replicate.

In other examples, the indication of an intended treatment area of the body part to be treated using the personal care device may be received in another way, for example from the personal care device itself. For example, it may be determined from a setting of the personal care device, or from the nature or type of attachment (e.g., cutting element or treatment element) fitted to the personal care device which area of the body part is it intended to treat.

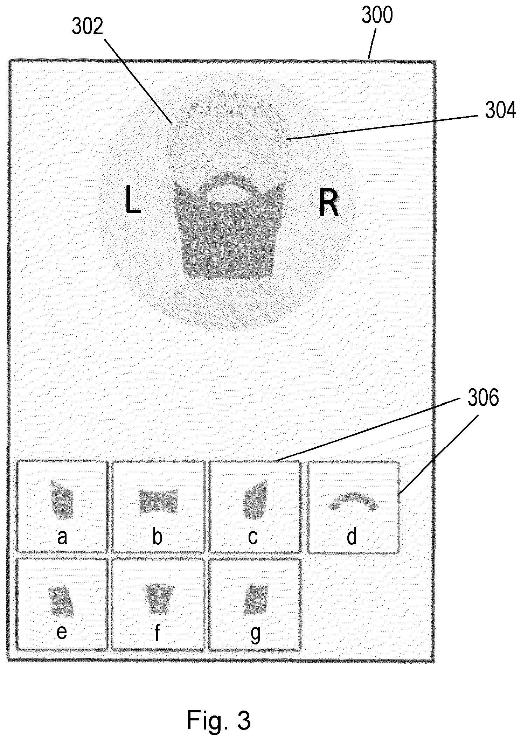

Fig. 3 is an illustration of an example of a user interface 300 on which a representation 302 of the body part 304 of a subject is displayed. The representation 302 may be a live representation of the subject (e.g., a live stream captured using a camera, or a reflection of the subject in an interactive mirror), a still image of the subject, an avatar representing the subject or a generic representation of the body part. In the example shown in Fig. 3, a generic segmented representation 302 of the head of a subject is shown. The user interface 300 also includes selectable icons 306, each corresponding to an area of the body part. The user may select one or more of the icons 306 to indicate the intended treatment area.

In another example, a user may provide the indication by drawing a freehand outline around the area that is intended to be treated. Alternatively, an image (e.g., a photograph) of the desired outcome of the personal care activity (e.g., a photograph of the subject with the desired facial hair) may be provided, for example by uploading the image, and the processor 202 may use a detection algorithm (e.g., a hair detection algorithm) to determine which areas of the body part are intended to be treated.

Referring again to Fig. 2, the processor 202 determines (step 104), based on the received indication received at step 102, geometrical properties 208 of the intended treatment area. In some embodiments, the geometrical properties 208 determined at step 104 may comprise dimensions of the intended treatment area. For example, the dimensions may be measurements in three dimensions, such as a width (W), a height (H) and a depth (D). Thus, in an example where the personal care activity is a shaving activity, the geometrical properties 208 (e.g., dimensions) of the shaving area may correspond to geometrical properties of the area when mapped to a three-dimensional version (e.g., a model) of the body part being shaved (e.g., the subject's head or face). In some embodiments, where an image of the intended treatment area is provided, geometrical properties 208 (e.g., dimensions) may be determined by mapping the image (e.g., a two-dimensional image) onto a generic three-dimensional model of the body part (e.g., a head), and determining the dimensions from the mapping. A more accurate determination may be made if multiple images of the intended treatment area are provided. The multiple images may be used to estimate the shape of the body part, rather than using a generic body part model, and the multiple images may then be mapped to the model to derive geometrical properties/dimensions. A further option is to estimate a three-dimensional body part shape, or estimate the depth of the body part, from a single image, and map the image to a model of the body part and derive geometrical properties/dimensions. In an example where the indication of the intended treatment area of the body part comprises an image of the intended treatment style or pattern body part, determining the geometrical properties 208of the intended treatment area may comprise determining the geometrical properties or dimensions from the intended treatment style or pattern in other words, the processor 202 may calculate the geometrical properties of the intended treatment area from the image of the intended treatment style or pattern provided by the user. In other examples, rather than determining dimensions of the intended treatment area, an indication of the shape (e.g., cylinder, sphere, cube, or the like) may be determined. In other examples, an indication of an angle and radius may be determined (e.g., part of a circle) to determine the size/extent of the intended treatment area.

Steps 102 and 104 may be performed prior to the personal care activity beginning. For example, a user may provide the indication of the intended treatment area in advance of starting the personal care activity. In some examples, a previously provided indication may be stored (e.g., a memory) and used by the processor 202 in the subsequent steps of the method 100.

During a personal care activity (e.g., a treatment session performed using the personal care device), a sensor (i.e., a first sensor 206) associated with the personal care device is configured to acquire data (i.e., first sensor data). The first sensor data acquired by the first sensor may be indicative of how the personal care device is moved during the treatment session. The first sensor may, for example, comprise an inertial measurement unit (IMU), an accelerometer, a displacement sensor, such as an optical displacement sensor, a motion or movement sensor, or a proximity sensor. An IMU may include one or more accelerometers, gyroscopes and/or magnetometers. In some examples, a personal care device may include multiple sensors, such as two or more of those discussed above, and this may improve the data acquired.

The processor 202 receives (step 106), from the first sensor 206, first sensor data associated with a location of the personal care device relative to the body part acquired during a treatment session performed using the personal care device. The received first sensor data may for example include a series of data points, each data point indicating a location of the personal care device relative to a reference point (e.g., the starting point of the personal care device). Thus, as the personal care device is moved during the treatment session, its location is recorded at a defined sampling rate, so over the course of the treatment session, a sequence of locations (e.g., a path of movement) is recorded. The nature of the data acquired by the first sensor 206 depends on the type of sensor used. For example, an IMU may measure and orientation and acceleration of the personal care device as it is moved and, from that data, the location of the personal care device relative to its starting position may be determined using known techniques.

The processor 202 predicts (step 108), based on the first sensor data received from the first sensor at step 106, a sequence of locations 210 of the personal care device relative to the body part. When acquiring the first sensor data, the first sensor 206 does not know which body part, or which area of the body part, is being treated. Therefore, based on the first sensor data, the processor 202 is able to predict a sequence of locations of the personal care device relative to its starting location, but the prediction does not take into account which body part is being treated, and whether the whole of the body part is being treated, or just a portion of the body part. Therefore, it is not possible to determine when the personal care device moves between different areas (e.g., segments) of the body part. For example, during a shaving activity, it is not possible first sensor data to determine when a shaving device moves from the cheek of the subject to the chin of the subject.

Thus, the processor 202 modifies (step 110) the sequence of predicted locations 210 based on the determined geometrical properties 208 of the intended treatment area. Modifying the sequence of predicted locations may involve mapping the sequence of predicted locations onto a three-dimensional model of the body part that has been treated. For example, a sequence of predicted locations from a shaving activity might be mapped onto a three-dimensional model of a person's head. In some embodiments, the three-dimensional model may be a generic model having the shape, size and dimensions of an average person's head. Such a generic body part may be given arbitrary dimensions of W=1 (e.g., 0 to 1), H=1 (e.g., 0 to 1) and D=1 (e.g., 0 to 1). Thus, sequence of predicted locations are scaled to fit within a cube of arbitrary dimensions 1 × 1 × 1.

In some embodiments, the total area covered by the sequence of predicted locations may be scaled (e.g., multiplied by a factor) based on the geometrical properties of the intended treatment area. For example, the sequence of predicted locations may be scaled to between 0 and 1 for each of three dimensions, x, y and z, and the scaling applied may correspond to the body part being treated (e.g., the head of the subject). If, at step 104, it is determined that the intended treatment area has a smaller area than the entire body part, then a scaling factor may be applied to reduce the area covered by the sequence of locations to match the geometrical properties of the intended treatment area. Thus, modifying the sequence of predicted locations may comprise rescaling the sequence of predicted locations based on the determined geometrical properties of the intended treatment area.

Example 1: Shaving an area of a head. The sequence of predicted locations are, by definition, contained in within a 0 to 1 (e.g., 1 × 1 × 1) cube. In this example, the scale of the treated area of the body part compared to the cube is HxWxD = 5x5x2 (in contrast to the scale of the entire head compared to this cube which is, for example, HxWxD = 30x20x15), and an origin point (0,0,0) is set at 15x7.5x10, instead of at 0x0x0 which is the case for the entire head. The sequence of predicted locations are re-scaled in a 0-1 cube to the actual dimensions (e.g., in cm), by multiplying them by the scale of the treated body part (i.e., 5x5x2 in this example), to get predictions on a cm scale. The scaled predictions are shifted to their actual locations relative to the model of the body part by adding the origin point. Those scaled and shifted predictions are then projected on the treated body part.

Example 2: Shaving an area of a head. In an alternative example, the scale of the sequence of predicted locations (i.e., the area of the sequence of predicted locations) may also be taken into account. In such examples, the actual dimensions of the predictions may be determined: for example, 0.8×0.7×0.4 (instead of 1×1×1 in Example 1 above). The scale of the treated area of the body part compared to the cube may then be determined (e.g., 5x5x2), and an origin point may be set (e.g., 15x7.5x10). The sequence of predicted locations are re-scaled to match the dimensions of the area of the body part that has been treated, by multiplying the area of the sequence of predicted locations by 5/0.8 × 5/0.7 × 2/0.4 so that the sequence of predicted locations end up fully spreading across the size of the treated body part. The scaled predictions are shifted to their actual locations relative to the model of the body part by adding the origin point 15x7.5x10. The scaled and shifted predictions can then be projected on the treated body part.

While, in some embodiments, a generic or standard sized body part may be used for scaling the sequence of predicted locations, in other embodiments, an area covered by the sequence of predicted locations (e.g., an area covered by a bounding box surrounding the sequence of predicted locations) may be estimated or predicted, and this may be scaled based on the geometrical properties (e.g., dimensions) of the intended treatment area.

During a personal care activity, a user may move a personal care device outside of the intended treatment area, for example by mistake or to enable them to view the intended treatment area. In some embodiments, if it is determined that the personal care device has been moved outside of the intended treatment area, then any predicted locations that are outside of the intended treatment area may be ignored. In some embodiments, responsive to determining that the sequence of predicted locations includes one or more locations that are outside of the intended treatment area, the sequence of predicted locations may be modified such that the sequence of modified locations is within the intended treatment area. In other words, if, following analysis of the path moved by the personal care device, it is determined that any locations in the sequence locations fell outside of the intended treatment area, then the area covered by the sequence of predicted locations may be scaled so that all of the locations in the sequence of predicted locations falls within the intended treatment area.

A result of performing the method 100 is that the sequence of predicted locations is modified to fit the intended treatment area as indicated by the user. Therefore, arbitrary motion data may be measured during a personal care activity, and this can be modified so that it applies to the intended treatment area of the body part, as indicated by the user.

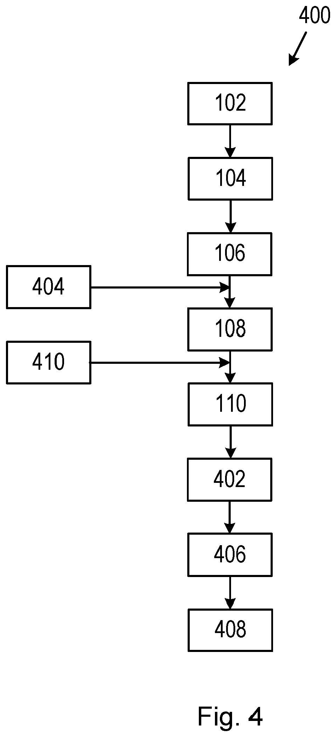

Fig. 4 is a flowchart of a further example of a method 400 for determining a location of a personal care device relative to a body part of a subject. At step 402, the method 400 may further comprise projecting the sequence of modified locations onto a representation of the intended treatment area. For example, a path during how the personal care device has moved during the treatment session may be projected onto a representation (e.g., an image, photograph, reflection, avatar or the like) of the intended treatment area of the body part. Projecting the sequence of modified locations onto the representation of the intended treatment area may be achieved using known projection techniques, including for example calculating the minimum Euclidean distance (e.g., relative to a representation/model of the body part). In other examples, a mesh model of the intended treatment area of the body part, for example using a 'point to the surface distance' technique, which will be understood by those skilled in the art. By projecting the sequence of modified locations onto a representation of the intended treatment area of the body part, sensor data acquired using the first sensor and/or the second sensor may be attributed to particular regions within the intended treatment area (e.g., left cheek, right cheek, chin, or the like). The method 400, which may comprise a computer-implemented method, may comprise steps of the method 100 discussed above.

At step 404, the method 400 may further comprise receiving, during a treatment session, from a second sensor associated with the personal care device, second sensor data relating to a treatment parameter associated with the personal care device. The second sensor may, for example comprise a pressure sensor or force sensor configured to measure a pressure applied by the personal care device onto the body part (e.g., onto the skin of the user), a timer configured to measure a time spent performing a personal care activity, a sensor configured to measure motion data indicating how the personal care device is moved during a personal care activity, or the like. In some embodiments, the second sensor data may be received from the first sensor 206. In some events, multiple second sensors may be provided, and a second sensor data may be received from two or more sensors.

The method 400 further comprises, at step 406, generating, for delivery to a recipient device, feedback relating to the treatment session, based on the first sensor data received from the first sensor and the second sensor data received from the second sensor. The first sensor data and the second sensor data may provide an indication of the extent to which the personal care device was moved over the intended treatment area during the treatment session, an indication of how the personal care device was moved during the treatment session, an indication of a pressure or force applied by the personal care device onto the body part of the user, an indication a speed of motion of the personal care device during the treatment session or the like. Based on the received first sensor data and second sensor data, an assessment of how well the user has performed the personal care activity during the treatment session may be made, and feedback may be provided to the user based on one or more parameters relating to the data measured with the first sensor and the second sensor. For example, the feedback may include an indication that the user moved the personal care device quicker while treating one side of the body part than the other side of the body part. In another example, the feedback may include an indication that the pressure applied to the body part during the treatment session was greater than normal, and that this could lead to skin irritation. In another example, the feedback may include an indication that the personal care device was moved linearly (e.g., generally in straight lines) during the treatment session, while it is recommended that the personal care device should be moved in a circular motion.

The feedback, in some embodiments, may include advice to enable the user to change one or more aspects of their use of the personal care device, to improve the effectiveness of its use in future treatment sessions. In other words, the feedback may be considered to be actionable feedback.

The recipient device to which the feedback is to be delivered may comprise the personal care device itself, or some other device with functionality enabling the feedback to be conveyed to a recipient, such as the user or subject. For example, the feedback may be delivered to a smart phone of the user (e.g., via an application), a tablet computer or an interactive mirror. The feedback may be provided in the form of a textual message displayed on a screen, for example "You pressed too hard during that session! Try applying less pressure next time." In other examples, the feedback may be provided in the form of one or more lights illuminating in different colours to indicate the quality of the treatment session.

In some embodiments, the feedback may be provided graphically or illustratively. For example, the method 400 may further comprise, at step 408, providing the generated feedback for presentation to a user on a representation of the body part. The representation of the body part may comprise a real-life likeness of the body part (e.g., based on a photograph or image of the subject's body part), a reflection of the body part (e.g., in an interactive mirror), an avatar, or a generic illustration of the body part. In an example in which the personal care activity is a shaving activity, a representation of the subject's face may be displayed, and different colours (e.g., red yellow and green) may be used to indicate how well the personal care activity was performed at different regions of the intended treatment area of the body part based on the first data and the second data (e.g., the motion, the pressure applied and/or the time spent). For example, a region coloured green may indicate that the personal care activity has been performed well in that region with regard to the data received from the first sensor and the second sensor, a region coloured yellow may indicate that the personal care activity has been performed reasonably well in that region, and that some improvement may benefit the user, and a region coloured red may indicate that the personal care activity has not been performed very well in region, and that improvement should be made.

The method 400 may, in some embodiments, further comprise, at step 410, applying a weight to each predicted location in the sequence of predicted locations based on a distance of the predicted location from the intended treatment area. For example, which may be applied using a weighted distance function. In some embodiments, a relatively low weights may be applied to a predicted location within the intended treatment area and a relatively large weight may be applied to a predicted location outside of the intended treatment area. The weight applied to a predicted location may increase with the distance outside of the intended treatment area, and a minimum weight may be applied to any predicted location falling within the intended treatment area.

In examples where weights have been applied to the predicted locations, modifying the sequence of predicted locations may be further based on the applied weights. By applying a minimum weighted distance technique, predicted locations that are a long way outside of the intended treatment area (therefore having a relatively large weight applied thereto) may be ignored or disregarded, and locations determined to be only slightly outside of the intended treatment area (therefore having a relatively small weight applied thereto) are taken into account. In this way, an event during the treatment session where the personal care device is inadvertently moved a long way outside of the intended treatment area, which may not have actually part of the treatment, may be disregarded.

In other embodiments, the weights may be applied in a different way. Once the sequence of predicted locations have been scaled, they may be projected onto a three-dimensional model of the entire body part (i.e., the treated and untreated areas). To decide where the projection ends up relative to the model of the body part, the point with the minimum weighted distance from the prediction to the three-dimensional model of the body part is taken into account. The weights for points within the treated area of the body part are set to a low value (e.g., to 1). The weights for points in the untreated area of the body part are set to a higher value (e.g., depending on the distance to the treated area of the body part). When projecting the sequence of predicted locations, the distance to each point in the three-dimensional model is computed and multiplied by the weight, and prediction with the minimum weighted distance is selected as a winner. As a result, the predictions that are far outside of the treated area will still be projected outside the treated area, whereas the rest will be projected inside the treated area. When providing feedback to the user, feedback in relation to points outside of the untreated area may be omitted.

A further example that takes into account the application of weights is shown below.

Example 3: Shaving an area of a head. First, the dimensions of the bulk of the sequence of predicted locations, excluding outliers, are determined (e.g., 0.8x0.7x0.4, instead of 1×1×1 as used in Example 1 above). The scale of the treated area of the body part compared to the cube is then determined, e.g., 5x5x2, and an origin point is set at 15×7.5×10. The sequence of predicted locations are re-scaled such that the bulk of the sequence of predicted locations match the dimensions of the treated area of the body part, by multiplying the predictions by 5/0.8 × 5/0.7 × 2/0.4 so that the bulk of the sequence of predicted locations end up fully spreading across the size of the treated body part. The scaled predictions are shifted to their actual locations relative to the model of the body part by adding the origin point 15x7.5x10. The scaled and shifted predictions can then be projected on the treated body part. In some embodiments, the outliers may be projected beyond (i.e., outside) the treated body part if that provides a better fit.

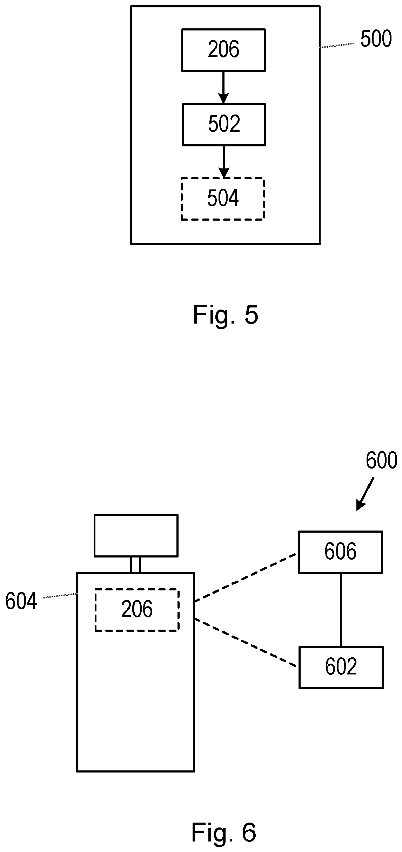

According to a further aspect, the present invention provides a personal care device. Fig. 5 is a schematic illustration of an example of a personal care device 500, which may be used to perform steps of the methods disclosed herein. The personal care device 500 comprises a first sensor 206 and a processor 502. The first sensor 206 is configured to measure first sensor data associated with a location of the personal care device relative to a body part to be treated. As discussed above, the first sensor 206 may comprise an inertial measurement unit (IMU), an accelerometer, a displacement sensor, a proximity sensor, or a similar component capable of determining motion data associated with the personal care device during treatment session. The processor 502, which may comprise or be similar to the processor 202 discussed above, is configured to perform steps of the methods 100, 400 disclosed herein. Specifically, the processor 502 is configured to receive an indication of an intended treatment area of the body part to be treated using the personal care device; determine, based on the received indication, geometrical properties of the intended treatment area; receive first sensor data from the first sensor during a treatment session performed using the personal care device; predict, based on the first sensor data received from the first sensor, a sequence of locations of the personal care device relative to the body part; and modify the sequence of predicted locations based on the determined geometrical properties of the intended treatment area.

In some embodiments, the personal care device 500 may further comprise a second sensor 504 configured to measure a treatment parameter associated with the personal care device. The second sensor 504 may for example, comprise a pressure sensor, a timer, a motion sensor, an IMU, or the like. The processor 502 may be configured to receive second sensor data from the second sensor 504 during the treatment session; and generate, for delivery to a recipient device, feedback relating to the treatment session based on first sensor data received from the first sensor 206 and second sensor data received from the second sensor 504.

The processor 502 may, in some embodiments, be configured to modify the sequence of predicted locations by rescaling the sequence of predicted locations such that the sequence of modified locations is within the intended treatment area. In other words, a path formed by the sequence of predicted locations of the personal care device during the treatment session may be scaled according to size of the intended treatment area.

According to a further aspect, the present invention provides a system. In the examples described above, the personal care device 500 includes the first sensor 206, the processor 502 and, where present, the second sensor 504. In these examples, the processing steps may be performed within the personal care device 500 itself. In other embodiments, the processing steps (e.g., the steps of the methods 100, 400 disclosed herein) may be performed using a processor located remote from the personal care device 500, such as a processor of a smart phone or a computing device in communication with the personal care device.

Fig. 6 is a schematic illustration of an example of a system 600 for determining a location of a personal care device relative to a body part of the subject. The system 600 comprises a processor 602, a personal care device 604 and a first sensor 606. The first sensor 606 is associated with the personal care device 604, and is configured to generate first sensor data associated with a location of the personal care device relative to the body part. The processor 602, which may be in communication with the personal care device 604 and/or the first sensor 606, is configured to receive an indication of an intended treatment area of the body part to be treated using the personal care device; determine, based on the received indication, geometrical properties of the intended treatment area; receive, from the first sensor, first sensor data generated by the first sensor during a treatment session performed using the personal care device; predict, based on the first sensor data received from the first sensor, a sequence of locations of the personal care device relative to the body part; and modify the sequence of predicted locations based on the determined geometrical properties of the intended treatment area. In some embodiments, the personal care device 604 may further comprise a second sensor (not shown) having the functionality of the second sensor 504 discussed above.

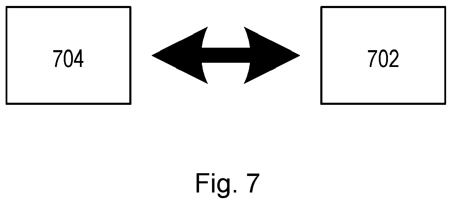

According to a further aspect, the present invention provides a computer program product. Fig. 7 is a schematic illustration of an example a processor to in communication with a machine-readable medium 704. According to various embodiments, a computer program product comprising a non-transitory computer-readable medium 704, the computer-readable medium having computer-readable code embodied therein, the computer-readable code being configured such that, on execution by a suitable computer or processor 702, the computer or processor is caused to perform steps of the methods 100, 400 disclosed herein.

The processor 202, 602, 702 can comprise one or more processors, processing units, multi-core processors or modules that are configured or programmed to control the components of the personal care device 500, 604 in the manner described herein. In particular implementations, the processor 202, 602, 702 can comprise a plurality of software and/or hardware modules that are each configured to perform, or are for performing, individual or multiple steps of the method described herein.

The term "module", as used herein is intended to include a hardware component, such as a processor or a component of a processor configured to perform a particular function, or a software component, such as a set of instruction data that has a particular function when executed by a processor.

It will be appreciated that the embodiments of the invention also apply to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to embodiments of the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g., Java interpreter instructions). Alternatively, one or more or all of the sub-routines may be stored in at least one external library file and linked with a main program either statically or dynamically, e.g., at run-time. The main program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer-implemented method (100) for determining a location of a personal care device relative to a body part of a subject, the method comprising:
receiving (102) an indication of an intended treatment area of the body part to be treated using the personal care device;
determining (104), based on the received indication, geometrical properties of the intended treatment area;
receiving (106), from a first sensor associated with the personal care device, first sensor data associated with a location of the personal care device relative to the body part acquired during a treatment session performed using the personal care device;
predicting (108), based on the first sensor data received from the first sensor, a sequence of locations of the personal care device relative to the body part; and
modifying (110) the sequence of predicted locations based on the determined geometrical properties of the intended treatment area.

2. A computer-implemented method (100) according to claim 1, wherein modifying the sequence of predicted locations comprises:
rescaling the sequence of predicted locations based on the determined geometrical properties of the intended treatment area.

3. A computer-implemented method (100) according to claim 1 or claim 2, wherein modifying the sequence of predicted locations comprises:
responsive to determining that the sequence of predicted locations includes one or more locations that are outside of the intended treatment area, modifying the sequence of predicted locations such that the sequence of modified locations is within the intended treatment area.

4. A computer-implemented method (100, 400) according to any of the preceding claims, further comprising:
projecting (402) the sequence of modified locations onto a representation of the intended treatment area.

5. A computer-implemented method (100, 400) according to any of the preceding claims, further comprising:
receiving (404), during a treatment session, from a second sensor associated with the personal care device, second sensor data relating to a treatment parameter associated with the personal care device;
generating (406), for delivery to a recipient device, feedback relating to the treatment session based on the first sensor data received from the first sensor and the second sensor data received from the second sensor.

6. A computer-implemented method (100, 400) according to claim 5, further comprising:
providing (408) the generated feedback for presentation to a user on a representation of the body part.

7. A computer-implemented method (100, 400) according to any of the preceding claims, wherein the indication of the intended treatment area of the body part comprises an indication of an area to be treated, or an area not to be treated, on a segmented representation of the body part displayed to the user.

8. A computer-implemented method (100, 400) according to any of the preceding claims, wherein the indication of the intended treatment area of the body part comprises an image of an intended treatment style or pattern on the body part; and
wherein determining the geometrical properties of the intended treatment area comprises determining the geometrical properties from the intended treatment style or pattern.

9. A computer-implemented method (100, 400) according to any of the preceding claims, further comprising:
applying (410) a weight to each predicted location in the sequence of predicted locations based on a distance of the predicted location from the intended treatment area;
wherein modifying the sequence of predicted locations is further based on the applied weights.

10. A personal care device (500) comprising:
a first sensor (206) configured to measure first sensor data associated with a location of the personal care device relative to a body part to be treated;
a processor (502) configured to:
receive an indication of an intended treatment area of the body part to be treated using the personal care device;
determine, based on the received indication, geometrical properties of the intended treatment area;
receive first sensor data from the first sensor during a treatment session performed using the personal care device;
predict, based on the first sensor data received from the first sensor, a sequence of locations of the personal care device relative to the body part; and
modify the sequence of predicted locations based on the determined geometrical properties of the intended treatment area.

11. A personal care device (500) according to claim 10, wherein the first sensor (206) comprises a sensor selected from a group comprising: an inertial measurement unit, IMU, an accelerometer, a displacement sensor, and a proximity sensor.

12. A personal care device (500) according to claim 10 or claim 11, further comprising:
a second sensor (504) configured to measure a treatment parameter associated with the personal care device;
wherein the processor (502) is configured to:
receive second sensor data from the second sensor during the treatment session; and
generate, for delivery to a recipient device, feedback relating to the treatment session based on first sensor data received from the first sensor and second sensor data received from the second sensor.

13. A personal care device (500) according to any of claims 10 to 12, wherein the processor (502) is configured to:
modify the sequence of predicted locations by rescaling the sequence of predicted locations such that the sequence of modified locations is within the intended treatment area.

14. A system (600) for determining a location of a personal care device relative to a body part of a subject, the system comprising:
a personal care device (604);
a first sensor (606) associated with the personal care device, the first sensor being configured to generate first sensor data associated with a location of the personal care device relative to the body part;
a processor (602) configured to:
receive an indication of an intended treatment area of the body part to be treated using the personal care device;
determine, based on the received indication, geometrical properties of the intended treatment area;
receive, from the first sensor, first sensor data generated by the first sensor during a treatment session performed using the personal care device;
predict, based on the first sensor data received from the first sensor, a sequence of locations of the personal care device relative to the body part; and
modify the sequence of predicted locations based on the determined geometrical properties of the intended treatment area.

15. A computer program product comprising a non-transitory computer-readable medium (704), the computer-readable medium having computer-readable code embodied therein, the computer readable code being configured such that, on execution by a suitable computer or processor (702), the computer or processor is caused to perform the method of any of the claims 1 to 9.
